(19) Europäisches Patentamt / European Patent Office / Office européen des brevets

(11)  EP 2 726 815 B1

# (12)  FASCICULE DE BREVET EUROPEEN

(45) Date de publication et mention de la délivrance du brevet:
**04.09.2019  Bulletin 2019/36**

(21) Numéro de dépôt: **12743484.3**

(22) Date de dépôt: **29.06.2012**

(51) Int Cl.:
*G01B 15/02* (2006.01)          *G01V 5/00* (2006.01)
*G01N 23/087* (2018.01)      *A61B 6/00* (2006.01)
*G01R 23/16* (2006.01)          *G01R 23/18* (2006.01)
*G06K 9/00* (2006.01)

(86) Numéro de dépôt international:
**PCT/FR2012/051510**

(87) Numéro de publication internationale:
**WO 2013/001247 (03.01.2013 Gazette 2013/01)**

(54) **PROCEDE ET DISPOSITIF D'IDENTIFICATION D'UN MATERIAU PAR ANALYSE SPECTRALE DE RAYONNEMENTS ELECTROMAGNETIQUES TRAVERSANT CE MATERIAU**

VERFAHREN UND VORRICHTUNG ZUM IDENTIFIZIEREN EINES MATERIALS DURCH SPEKTRALANALYSE VON ELEKTROMAGNETISCHEM STRAHLUNGSDURCHGANG DURCH DIESES MATERIAL

METHOD AND DEVICE FOR IDENTIFYING A MATERIAL BY THE SPECTRAL ANALYSIS OF ELECTROMAGNETIC RADIATION PASSING THROUGH SAID MATERIAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.06.2011  FR 1102070**

(43) Date de publication de la demande:
**07.05.2014  Bulletin 2014/19**

(73) Titulaire: **Commissariat à l'Énergie Atomique et aux Énergies Alternatives 75015 Paris (FR)**

(72) Inventeurs:
• **BELDJOUDI, Guillaume**
  **F-69520 Grigny (FR)**
• **REBUFFEL, Véronique**
  **F-38700 Corenc (FR)**
• **RINKEL, Jean**
  **91440 Bures-sur-Yvette (FR)**

(74) Mandataire: **Bonnet, Michel**
  **Cabinet Bonnet**
  **93, rue Réaumur - Boîte 10**
  **75002 Paris (FR)**

(56) Documents cités:
WO-A1-01/50112          FR-A1- 2 953 603
US-A1- 2007 108 379      US-A1- 2008 298 544

• MEYER K ET AL: "Qualitative and quantitative mixture analysis by library search: infrared analysis of mixtures of carbohydrates", ANALYTICA CHIMICA ACTA, ELSEVIER, AMSTERDAM, NL, vol. 281, no. 1, 1 septembre 1993 (1993-09-01), pages 161-171, XP026592982, ISSN: 0003-2670, DOI: 10.1016/0003-2670(93)85350-S [extrait le 1993-09-01]

EP 2 726 815 B1

**Description**

La présente invention concerne un procédé d'identification d'un matériau par analyse spectrale de rayonnements électromagnétiques aptes à traverser ce matériau. Elle concerne également un dispositif et un programme d'ordinateur correspondants.

Elle concerne entre autres les domaines de l'analyse spectrale par rayons X et gamma. Cette analyse est par exemple utilisée dans les applications de contrôle non destructif, notamment de contrôle de bagages par radiographie en défilement pour la détection de matériaux explosifs dans les aéroports. L'application de contrôle de bagages par défilement est en particulier très exigeante en termes de rapidité (la vitesse de défilement des bagages impose de faire une mesure de l'énergie des photons transmis à travers les bagages sur un temps généralement court, quelques ms, avec un flux de photons incident pouvant être élevé, quelques dizaines de Mphotons/mm$^2$/s, pour garder une statistique suffisante), de précision et de fiabilité (pour des raisons évidentes de sécurité).

L'invention s'applique plus particulièrement à un procédé d'identification comportant les étapes suivantes :

- mesure d'un spectre d'un rayonnement électromagnétique émis à travers le matériau à identifier,
- détermination d'au moins une bande d'énergies, dite bande de mesure, et de coefficients spectraux d'une fonction de comparaison dans cette bande de mesure, à partir du spectre mesuré,
- estimation, à l'aide des coefficients spectraux déterminés, de la nature et/ou de l'épaisseur du matériau à identifier sur la base d'un ensemble de paramètres spectraux de référence relatifs à des matériaux et/ou épaisseurs de référence, ces paramètres spectraux de référence étant définis pour chaque matériau et/ou épaisseur de référence dans au moins une bande d'énergies dite bande de référence.

D'une façon générale, par « spectre » d'un rayonnement électromagnétique, on entend un ensemble de valeurs de l'intensité de ce rayonnement électromagnétique en fonction de l'énergie photonique.

Par « fonction de comparaison » liée à un matériau à identifier on entend une fonction comparant, à une énergie donnée ou sur au moins une bande d'énergies donnée, l'intensité du rayonnement électromagnétique transmis par le matériau à l'intensité du rayonnement électromagnétique incident au matériau.

Plus précisément, cette comparaison est généralement réalisée par un ratio, si bien que la fonction de comparaison est obtenue en effectuant, à une énergie donnée ou sur au moins une bande d'énergies donnée, un rapport entre l'intensité du rayonnement transmis et l'intensité du rayonnement incident.

Ainsi, si I(E) et I$_0$(E) désignent respectivement le nombre de photons transmis par le matériau et le nombre de photons incidents au matériau par unité de temps à l'énergie E ou dans la bande d'énergies E, la fonction de comparaison TR(E) peut prendre la forme suivante :

$$TR(E) = \frac{I(E)}{I_0(E)} . \tag{1}$$

Sous cette forme, la fonction de comparaison peut être désignée par le terme de fonction de transmission.

Par ailleurs, si $\mu$(E) désigne le coefficient d'atténuation linéaire du matériau à l'énergie E ou dans la bande d'énergies E et si d désigne l'épaisseur de matériau traversée par le rayonnement, on sait que :

$$I(E) = I_0(E)e^{-\mu(E)d} .$$

Ainsi, afin d'obtenir une fonction de comparaison linéairement dépendante du coefficient d'atténuation linéaire du matériau, il peut être avantageux d'exprimer la transmission des photons dans le matériau en utilisant le logarithme népérien du ratio précédemment décrit. Dans ce cas, la fonction de comparaison TR(E) peut prendre la forme suivante :

$$TR(E) = -\ln\left(\frac{I(E)}{I_0(E)}\right) . \tag{2}$$

Sous cette forme, la fonction de comparaison est usuellement désignée par le terme de fonction d'atténuation.

Par conséquent, dans cette demande de brevet, le terme de fonction de comparaison couvre à la fois :

- une fonction de transmission obtenue à partir d'un rapport entre l'intensité du rayonnement transmis par le matériau et l'intensité du rayonnement incident au matériau à une énergie donnée ou sur au moins une bande d'énergies donnée, telle que par exemple celle définie par l'équation (1), et
- une fonction d'atténuation obtenue à partir du logarithme népérien du rapport défini dans l'alinéa précédent, telle que par exemple celle définie par l'équation (2).

Les grandeurs $I(E)$ et $I_0(E)$, qui désignent respectivement l'intensité du rayonnement transmis et l'intensité du rayonnement incident, ont été plus précisément définies précédemment comme des flux représentatifs respectivement d'un nombre de photons transmis par le matériau et d'un nombre de photons incidents au matériau par unité de temps à l'énergie E ou dans la bande d'énergies E, mais elles peuvent également être définies comme des débits de fluence (nombre de photons par unités de temps et de surface) ou comme des nombres de photons détectés durant un temps déterminé. Dans la suite de la description, elles seront définies comme des flux. Bien sûr, elles doivent être homogènes, c'est-à-dire représenter une grandeur de même type, pour que la fonction de comparaison soit sans unité.

Les grandeurs $I(E)$ et $I_0(E)$ sont concrètement mesurées à l'aide d'un système de détection situé sur le trajet du rayonnement électromagnétique :

- en présence du matériau à identifier entre la source d'émission du rayonnement et le système de détection pour l'estimation de $I(E)$, et
- en l'absence du matériau à identifier, ou par calcul si l'on connaît les paramètres d'émission de la source, pour l'estimation de $I_0(E)$.

Un système de détection connu est par exemple constitué de plusieurs détecteurs scintillateurs n'ayant pas de fonction spectrométrique. De tels détecteurs sont superposés les uns aux autres, des écrans filtrants intercalaires pouvant être placés entre deux détecteurs successifs. Généralement, on utilise deux détecteurs selon la dénomination "capteurs Sandwich" : le premier détecteur est de faible volume, de sorte qu'il absorbe principalement les photons de faible énergie, et le second détecteur, placé après le premier sur le trajet du rayonnement électromagnétique, est de volume plus important, de sorte qu'il absorbe principalement les photons d'énergie élevée. Ainsi, en utilisant ces premier et second détecteurs, on mesure respectivement une intensité d'une composante de basse énergie et une intensité d'une composante de haute énergie du rayonnement électromagnétique, ces deux intensités formant deux composantes d'un spectre du rayonnement. En effectuant cette mesure en présence et en l'absence du matériau à identifier, on obtient la mesure d'une fonction de comparaison à deux valeurs, en l'occurrence deux coefficients spectraux de transmission ou d'atténuation sur deux bandes d'énergies formant la bande de mesure.

Un autre système de détection connu et plus récent comporte par exemple un détecteur spectrométrique, notamment par exemple de rayons X, permettant d'obtenir la mesure d'un spectre sur toute une gamme d'énergies et donc la détermination de coefficients spectraux d'une fonction de comparaison sur au moins une partie de cette gamme d'énergies formant la bande de mesure.

Quel que soit le système de détection utilisé, la mesure permet ainsi d'obtenir une pluralité de coefficients spectraux d'une fonction de comparaison dans la bande de mesure.

A l'aide du système de détection, il est également connu de procéder à un calibrage par la détermination de fonctions de comparaisons d'objets étalons, différant les uns des autres par la nature de leur matériau et/ou leur épaisseur. On obtient ainsi de même pour chaque objet étalon, de matériau et épaisseur donnés, des paramètres spectraux de référence, en particulier des coefficients spectraux de transmission ou d'atténuation de référence, dans au moins une bande d'énergie dite bande de référence propre à l'objet étalon considéré.

Pour estimer la nature et/ou l'épaisseur du matériau à identifier, une solution immédiate est de calculer, à l'aide par exemple d'une fonction de distance prédéterminée, les coefficients spectraux de référence les plus proches des coefficients spectraux déterminés pour le matériau à identifier. On considère alors que le matériau à identifier présente les caractéristiques (nature du matériau et/ou épaisseur) de l'objet étalon le plus proche. D'une façon plus générale, l'estimation de la nature et/ou de l'épaisseur du matériau à identifier se fait par une confrontation des coefficients spectraux déterminés par la mesure avec les paramètres spectraux de référence. Il est donc nécessaire pour cela de comparer les coefficients aux paramètres de référence dans des bandes d'énergies communes. Il convient alors de ne considérer que les coefficients correspondant à des énergies communes à la bande de mesure et à toutes les bandes de référence, c'est-à-dire à l'intersection de la bande de mesure et de toutes les bandes de référence.

Le problème de cette estimation est qu'elle doit ainsi se faire dans une gamme d'énergies éventuellement très restreinte, d'autant plus que la mesure par spectrométrie peut aboutir à une absence de photons transmis dans certains canaux d'énergies, rendant ces canaux inexploitables pour l'estimation. Comme en plus ce problème se pose à la fois lors de la mesure portant sur le matériau à identifier et lors du calibrage, la bande d'énergies commune exploitée pour l'identification peut être très réduite. En particulier, les objets étalons de forte épaisseur sont les plus atténuants et donc les plus responsables de la restriction de la bande d'énergie commune, ce qui rend les matériaux de faible épaisseur plus difficiles à identifier. De même, des objets étalons dans des matériaux tels que le fer ou le plomb induisent des gammes d'énergies restreintes et rendent difficiles l'identification de faibles épaisseurs de matériaux légers tels que les plastiques.

Enfin, un bruit photonique existe sur les mesures d'atténuations de sorte que la détermination de la gamme d'énergie commune n'est pas aisée puisqu'elle est sujette aux variations de bruit photonique.

On connaît aussi par le document "Qualitative and quantitative mixtures analysis by library search: infrared analysis of mixtures of carbohydrates", K. Meyer et al, Analytica Chimica Acta, vol. 281, pages 161-171, 01/09/1993, un procédé d'analyse de mélanges d'hydrates de carbone par spectrométrie infrarouge.

Il peut ainsi être souhaité de prévoir un procédé d'identification d'un matériau, par analyse spectrale de rayonnements électromagnétiques aptes à traverser ce matériau, qui permette de s'affranchir d'au moins une partie des problèmes et contraintes précités.

L'invention a donc pour objet un procédé d'identification d'un matériau, tel que revendiqué dans la revendication 1.

Ainsi, cette estimation en au moins deux phases distinctes comportant une sélection préalable de candidats possibles permet de procéder à l'estimation finale de la nature et/ou de l'épaisseur du matériau à identifier sur la base d'une bande d'énergies commune plus importante (intersection de la bande de mesure et des bandes de référence des candidats possibles) que dans l'état de la technique (intersection de la bande de mesure et de toutes les bandes de référence), tout en prévoyant lors de chaque phase de confronter les coefficients spectraux déterminés par la mesure avec au moins une partie des paramètres spectraux de référence. La fiabilité du procédé d'identification s'en trouve améliorée.

De façon optionnelle, la sélection préalable des candidats possibles se fait :

- soit dans au moins une bande d'énergies commune à toutes les bandes de référence et à la bande de mesure,
- soit dans au moins une bande d'énergies commune à une partie prédéterminée des bandes de référence et à la bande de mesure.

De façon optionnelle également, les matériaux et/ou épaisseurs de référence comportent une pluralité de matériaux de référence à une pluralité d'épaisseurs de référence chacun.

De façon optionnelle également, l'estimation comporte les étapes suivantes, exécutées pour chaque matériau de référence :

- sélection d'une pluralité d'épaisseurs de référence pour ce matériau de référence, dites candidates possibles, par confrontation des coefficients spectraux déterminés avec les paramètres spectraux de référence dans au moins une bande d'énergies commune aux bandes de référence de chaque épaisseur de référence pour ce matériau de référence et à la bande de mesure,
- sélection de deux épaisseurs de référence consécutives pour ce matériau de référence par confrontation des coefficients spectraux déterminés avec les paramètres spectraux d'au moins une partie des candidats possibles, dans au moins une bande d'énergies commune aux bandes de référence de ladite au moins une partie des candidats possibles et à la bande de mesure,
- estimation d'une épaisseur optimale pour ce matériau de référence par confrontation des coefficients spectraux déterminés avec des paramètres spectraux interpolés à partir des paramètres spectraux relatifs aux deux épaisseurs de référence consécutives sélectionnées, dans au moins une bande d'énergies commune aux bandes de référence des deux épaisseurs de référence consécutives sélectionnées et à la bande de mesure.

De façon optionnelle également, l'estimation de la nature et/ou de l'épaisseur du matériau à identifier comporte la sélection d'une épaisseur optimale, parmi l'ensemble des épaisseurs optimales associées chacune à un matériau de référence, sur la base d'une optimisation d'un critère évalué dans au moins une bande d'énergies commune à l'ensemble des bandes d'énergies dans lesquelles les épaisseurs optimales ont été estimées.

De façon optionnelle également, les paramètres spectraux de référence relatifs à des matériaux et/ou épaisseurs de référence sont des paramètres statistiques de densités de probabilité, chaque matériau et/ou épaisseur de référence étant ainsi associé à une densité de probabilité permettant le calcul d'une probabilité de mesurer des coefficients spectraux en présence de ce matériau et/ou épaisseur de référence dans sa bande de référence.

De façon optionnelle également, la confrontation des coefficients spectraux déterminés avec les paramètres spectraux de référence relatifs à un matériau et/ou une épaisseur de référence comporte l'estimation d'une valeur que prend la densité de probabilité de ce matériau et/ou cette épaisseur de référence lorsque lui sont appliqués les coefficients spectraux déterminés.

De façon optionnelle également, la confrontation des coefficients spectraux déterminés avec les paramètres spectraux de référence relatifs à un matériau et/ou épaisseur de référence comporte l'estimation d'une probabilité a posteriori conditionnelle d'être en présence de ce matériau et/ou épaisseur de référence connaissant les coefficients spectraux déterminés, sur la base du produit d'une probabilité a posteriori conditionnelle de mesurer les coefficients spectraux déterminés en présence de ce matériau et/ou épaisseur de référence et d'une probabilité a priori d'être en présence de ce matériau et/ou épaisseur de référence, par application du théorème de Bayes.

L'invention a également pour objet un dispositif d'identification d'un matériau, tel que revendiqué dans la revendication 9.

Enfin, l'invention a également pour objet un programme d'ordinateur téléchargeable depuis un réseau de communication et/ou enregistré sur un support lisible par ordinateur et/ou exécutable par un processeur, comprenant des instructions pour l'exécution des étapes d'un procédé d'identification d'un matériau selon l'invention, lorsque ledit programme est exécuté sur un ordinateur.

L'invention sera mieux comprise à l'aide de la description qui va suivre, donnée uniquement à titre d'exemple et faite en se référant aux dessins annexés dans lesquels :

- la figure 1 représente schématiquement la structure générale d'un dispositif d'identification d'un matériau par analyse spectrale de rayonnements électromagnétiques aptes à traverser ce matériau, selon un mode de réalisation de l'invention,
- la figure 2 illustre les étapes successives d'un procédé de calibrage mis en oeuvre par le dispositif de la figure 1, selon un mode de réalisation de l'invention,
- la figure 3 illustre les étapes successives d'un procédé d'identification mis en oeuvre par le dispositif de la figure 1, selon un premier mode de réalisation de l'invention,
- les figures 4 et 5 illustrent les fonctionnements respectifs de certaines étapes du procédé d'identification de la figure 3, et
- la figure 6 illustre les étapes successives d'un procédé d'identification selon un second mode de réalisation de l'invention.

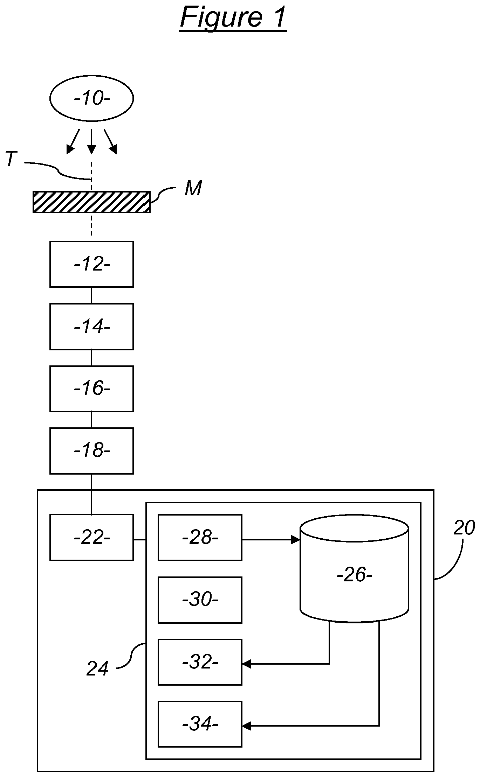

Le dispositif d'identification représenté schématiquement sur la figure 1 comporte un émetteur 10 de rayonnement électromagnétique, par exemple une source de rayons X ou gamma. S'il s'agit d'une source de rayons X, l'émetteur 10 comporte par exemple une anode en tungstène, un filtre de 3 mm d'aluminium et est alimenté sous une tension de 115 kV. Les paramètres classiques d'intensité et de temps sont par exemple fixés pour obtenir une fluence de $7,8. 10^4$ photons/pixel.

Sur le trajet T du rayonnement émis, le dispositif d'identification comporte en outre un détecteur 12, par exemple un détecteur spectrométrique. Ce détecteur permet de mesurer un spectre d'un rayonnement transmis à travers un matériau à identifier M, ce dernier étant disposé entre l'émetteur 10 et le détecteur 12 sur le trajet T.

Plus précisément, le détecteur 12 est par exemple un capteur à conversion directe, notamment en matériau semi-conducteur (CdTe, CdTe:Cl, CdTe:ln, CdZnTe, CdMnTe, HgI2, AsGa, Si, TlBr, etc.). Ce capteur est muni de deux électrodes aux bornes desquelles une impulsion de signal traduit une interaction d'un rayonnement ou d'un photon avec le matériau du capteur et la création d'un nuage de charges électroniques (typiquement de l'ordre de 10000 électrons à quelques dizaines de milliers d'électrons pour un photon X de 60 keV), dans le matériau du capteur, qui résulte de cette interaction. Les charges sont ensuite captées par les deux électrodes entre lesquelles est établie une différence de potentiel requise. Si la collecte est complète, l'intégrale de l'impulsion mesurée est proportionnelle à l'énergie déposée par la particule incidente.

En sortie du détecteur spectrométrique 12, le dispositif d'identification comporte en outre un préamplificateur de charges 14, un amplificateur 16 et un convertisseur analogique/numérique 18 pour la fourniture de valeurs numériques du spectre mesuré par le détecteur 12 à un calculateur 20.

Le calculateur 20 est par exemple un ordinateur classique comportant un processeur 22 associé à une ou plusieurs mémoire(s) identifiée(s) par la référence générique 24. La mémoire 24 stocke une base de données 26 de paramètres spectraux de référence relatifs à des matériaux et épaisseurs de référence, ces paramètres spectraux de référence étant définis pour chaque matériau et épaisseur de référence dans au moins une bande d'énergies dite bande de référence.

La mémoire 24 stocke en outre un ou plusieurs programmes d'ordinateurs 28, 30, 32, 34 constitués de séquences d'instructions permettant, lorsqu'elles sont exécutées par le processeur 22, de réaliser les opérations suivantes :

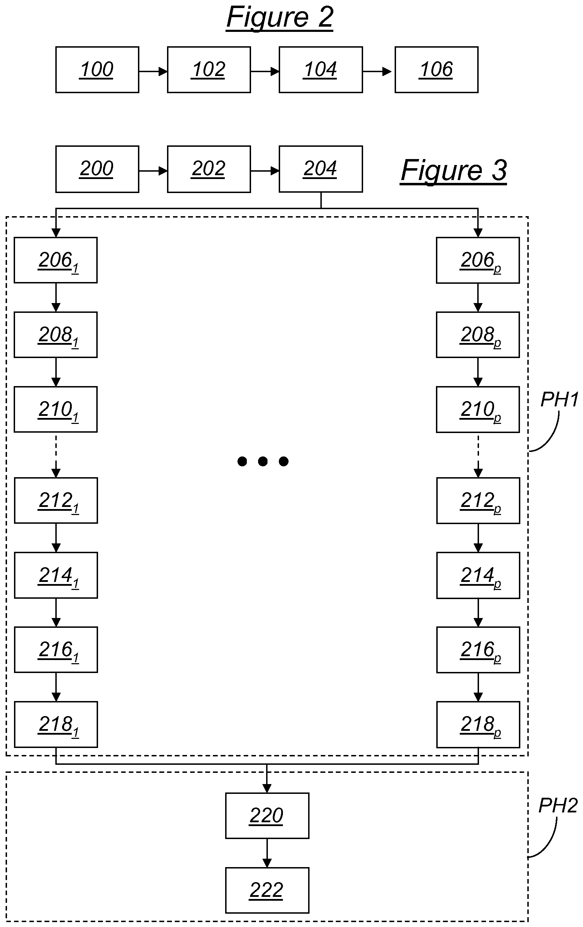

- calculer les paramètres spectraux de référence de la base de données 26, lors de la mise en oeuvre d'un procédé de calibrage à l'aide d'un ensemble d'objets étalons de matériaux et d'épaisseurs connus : ce procédé de calibrage, qui sera détaillé en référence à la figure 2, est mis en oeuvre par le programme 28,
- déterminer au moins une bande d'énergies, dite bande de mesure et des coefficients spectraux d'une fonction de comparaison dans cette bande de mesure, à partir de valeurs numériques d'un spectre mesuré fournies au calculateur : cette détermination des coefficients spectraux dans la bande de mesure est réalisée par le programme 30,

- sélectionner une pluralité d'objets étalons, dits candidats possibles, à partir d'une confrontation des coefficients spectraux déterminés par le programme 30 avec au moins une partie des paramètres spectraux de référence : cette sélection préalable est réalisée en une ou plusieurs itérations par le programme 32, et
- estimer la nature et/ou l'épaisseur du matériau à identifier à partir d'une confrontation des coefficients spectraux déterminés par le programme 30 avec les paramètres spectraux d'au moins une partie des candidats possibles, dans au moins une bande d'énergies commune aux bandes de référence de ladite au moins une partie des candidats possibles et à la bande de mesure : cette estimation est réalisée par le programme 34.

Le procédé d'identification correspondant à l'exécution des programmes 30, 32 et 34 sera détaillé en référence à la figure 3, selon un mode de réalisation possible de l'invention.

On notera par ailleurs que les programmes d'ordinateurs 28, 30, 32, 34 sont présentés comme distincts, mais cette distinction est purement fonctionnelle. Ils pourraient tout aussi bien être regroupés en un ou plusieurs logiciels. Leurs fonctions pourraient aussi être micro programmées dans des circuits intégrés dédiés.

L'identification d'un matériau inconnu M consiste à déterminer par exemple la nature de ce matériau et son épaisseur à partir d'une confrontation des coefficients spectraux issus d'une mesure de ce matériau inconnu avec les paramètres spectraux de référence de la base de données 26 portant sur les objets étalons. Selon différents modes de réalisation possibles, les paramètres spectraux de référence peuvent être des coefficients spectraux directement comparables aux coefficients spectraux mesurés pour le matériau inconnu M ou bien des paramètres statistiques portant sur de tels coefficients, notamment des paramètres de densités de probabilités. Ainsi, dans le premier cas, la confrontation peut consister en une comparaison des coefficients mesurés avec les coefficients spectraux de référence, notamment par un calcul de distance. Dans le second cas, la confrontation peut consister en une recherche de maximum, par exemple parmi les valeurs que prennent les densités de probabilités de référence aux valeurs mesurées ou parmi des probabilités explicitement calculées, par inférence bayésienne notamment, sur la base des valeurs mesurées. Dans tous les cas, la confrontation se ramène à l'optimisation d'une fonction de coût, cette dernière étant paramétrée par les paramètres spectraux de référence et étant évaluée aux valeurs prises par les coefficients spectraux issus de la mesure.

Les paramètres spectraux de référence peuvent être a priori connus et stockés dans la base de données 26. Ils peuvent également être obtenus par une succession de mesures et de calculs illustrés sur la figure 2.

**Détermination des paramètres spectraux de référence**

Le procédé de calibrage illustré sur cette figure comporte une première étape 100 d'acquisition de spectres sans qu'aucun objet ne soit disposé entre l'émetteur 10 et le détecteur 12. Cette acquisition est répétée plusieurs fois, par exemple 1000 fois, de manière à obtenir un spectre moyen $I_0$ d'un rayonnement électromagnétique émis par l'émetteur 10. Chaque spectre acquis en l'absence d'objet entre l'émetteur 10 et le détecteur 12, puis numérisé par le convertisseur 18, est un histogramme indiquant un nombre de photons reçus à chaque énergie, dans une gamme d'énergies discrètes prédéterminée, par exemple entre 0 et 150 keV.

Ces spectres sont moyennés sur un grand nombre d'acquisitions pour minimiser l'effet du bruit photonique. Il peut être ensuite imposé une valeur seuil en termes de nombre de photons moyens reçus à chaque énergie, par exemple 5, en deçà de laquelle les valeurs de ce spectre moyen $I_0$ ne sont pas prises en compte. Le spectre moyen d'émission $I_0$ est donc ainsi finalement retenu dans au moins une bande d'énergies, dite bande d'émission $B_0$. Dans cette bande d'émission $B_0$, on remarque notamment qu'il n'y a pas de valeurs nulles du spectre moyen $I_0$, ce qui est important pour pouvoir calculer les fonctions de comparaisons des objets étalons et des matériaux à identifier.

On notera que les acquisitions successives sont réalisées par le détecteur 12, par exemple sur commande du calculateur 20, alors que les calculs du spectre moyen $I_0$ et de la bande d'émission $B_0$ sont effectués grâce à une exécution du programme 28 par le processeur 22.

A partir de l'étape 102 suivante, le procédé de calibrage est appliqué pour chaque objet étalon dont on connaît le matériau et l'épaisseur. Pour un objet étalon Mref de matériau et d'épaisseur connus, l'étape 102 consiste à acquérir, comme à l'étape 100, plusieurs spectres, par exemple 1000, de manière à obtenir un spectre moyen I d'un rayonnement électromagnétique transmis par l'objet étalon Mref. Comme précédemment, chaque spectre acquis en présence de l'objet étalon Mref, puis numérisé par le convertisseur 18, est un histogramme indiquant un nombre de photons reçus à chaque énergie, dans la même gamme d'énergies discrètes prédéterminée.

Ces spectres sont moyennés sur un grand nombre d'acquisitions pour minimiser de nouveau l'effet du bruit photonique. Il peut être ensuite imposé une valeur seuil en termes de nombre de photons moyens reçus à chaque énergie, par exemple 5, en deçà de laquelle les valeurs de ce spectre moyen I ne sont pas prises en compte. Le spectre moyen I est donc finalement retenu dans au moins une bande d'énergies, dite bande de transmission B. Dans cette bande de transmission B, on remarque également qu'il n'y a pas de valeurs nulles du spectre moyen I, ce qui est préférable parce que le fait de ne mesurer aucun photon transmis dans un canal donné n'apporte pas d'information pertinente sur l'objet considéré : tout au plus cela signifie-t-il que l'objet est suffisamment atténuant dans ce canal d'énergies pour absorber tous les photons.

On notera de même que les acquisitions successives sont réalisées par le détecteur 12, par exemple sur commande du calculateur 20, alors que les calculs du spectre moyen I et de la bande de transmission B sont effectués grâce à une exécution du programme 28 par le processeur 22.

Au cours d'une étape 104, réalisée sur exécution du programme 28, l'intersection entre la bande d'émission $B_0$ et la bande de transmission B pour l'objet étalon Mref est déterminée. Elle définit la bande de référence B(Mref) pour l'objet étalon Mref. Cette bande de référence est constituée d'une pluralité de canaux, chaque canal correspondant à une plage d'énergies. En particulier, une plage d'énergie peut être représentée par une valeur d'énergie discrète de la gamme d'énergies discrètes des spectres fournis par le convertisseur 18.

Ensuite, au cours d'une étape 106 d'établissement d'une fonction de comparaison, réalisé sur exécution du programme 28, à chaque valeur d'énergie discrète pour laquelle le calcul est réalisable dans la bande de référence et pour chaque spectre acquis à l'étape 102, un calcul de coefficient de comparaison est réalisé, par exemple sur la base de l'équation (1) (coefficient de transmission) ou de l'équation (2) (coefficient d'atténuation). On obtient ainsi autant de fonctions de comparaisons que de spectres mesurés.

Enfin, au cours d'une dernière étape 108, réalisée sur exécution du programme 28, les paramètres spectraux de référence sont déterminés à partir des fonctions de comparaisons précédemment établies, puis stockés dans la base de données 26.

Selon un premier mode de réalisation possible, les paramètres spectraux de référence sont des coefficients spectraux obtenus, dans chaque canal de la bande de référence, en moyennant les valeurs des fonctions de comparaisons précédemment établies. Autrement dit, on réalise une moyenne statistique de chaque fonction de comparaison, et on détermine les coefficients spectraux à partir des valeurs, dans différents canaux d'énergie, de cette fonction de comparaison. Ainsi, en pratique, à chaque valeur d'énergie discrète dans la bande de référence B(Mref) de l'objet étalon considéré, la moyenne de tous les coefficients de comparaison correspondants calculés à l'étape 106 est retenue en tant que coefficient spectral de référence pour le canal correspondant à cette énergie discrète. Ainsi, si la bande de référence B(Mref) comporte N(Mref) canaux, c'est-à-dire en fait N(Mref) valeurs d'énergie discrètes, N(Mref) coefficients spectraux de référence sont calculés et enregistrés dans la base de données 26 pour l'objet étalon Mref. On notera que puisque les coefficients de comparaison n'ont peut-être pas pu être tous calculés à l'étape 106, du fait d'une absence possible de photons mesurés pour certaines acquisitions à certaines énergies, les valeurs moyennes que constituent les coefficients spectraux de référence ne sont pas toutes calculées sur le même nombre de valeurs, mais cela ne pose pas de problème particulier, si ce n'est un éventuel léger biais largement atténué par le nombre d'acquisitions. En outre, de façon optionnelle, il est également possible de ne pas tenir compte des coefficients de valeur nulle dans le calcul des moyennes, si l'on souhaite considérer que ces valeurs nulles (pas de photon mesuré dans le canal correspondant) ne sont pas significatives.

Comme indiqué précédemment, selon d'autres modes de réalisation possibles, les paramètres spectraux de référence de l'objet étalon Mref peuvent être des paramètres statistiques, notamment des paramètres d'une densité de probabilité permettant le calcul d'une probabilité de mesurer des coefficients spectraux en présence de l'objet étalon Mref dans sa bande de référence B(Mref). A titre d'exemple non limitatif, sur la base d'un modèle de loi multinormale de dimension N(Mref) pouvant se simplifier en N(Mref) lois normales indépendantes opérant chacune dans l'un des canaux de la bande de référence B(Mref) pour la modélisation statistique du coefficient spectral de référence correspondant, les paramètres spectraux de référence sont obtenus, dans chaque canal de la bande de référence, en établissant :

- la moyenne des valeurs des fonctions de comparaisons précédemment établies (en excluant éventuellement les valeurs nulles), et
- l'écart-type des valeurs des fonctions de comparaisons précédemment établies (en excluant éventuellement les valeurs nulles).

Ainsi, N(Mref) moyennes spectrales de référence et N(Mref) écarts-types spectraux de référence sont calculés et enregistrés dans la base de données 26 pour l'objet étalon Mref.

Le procédé de calibrage détaillé ci-dessus peut être exécuté pour p matériaux de référence différents et, pour chacun de ces p matériaux de référence, pour un nombre variable d'épaisseurs de référence. A titre d'exemple, dans une application de détection de matériaux plastiques, il peut être utilisé p=3 matériaux de référence différents, à savoir le Polyéthylène (PE), le Polyoxyméthylène (POM) et le Polyfluorure de vinylidène (PVDF), et, pour chacun de ces trois matériaux, 50 épaisseurs variant de 1 à 50 mm par pas de 1 mm. On obtient ainsi une base de données 26 de paramètres spectraux de référence relatifs à 150 objets étalons.

**Identification d'un matériau inconnu**

Le procédé d'identification d'un matériau inconnu M va maintenant être détaillé en référence à la figure 3. Il consiste par exemple à déterminer sa nature et son épaisseur à partir d'une confrontation des coefficients spectraux issus d'une mesure de ce matériau avec les paramètres spectraux de référence précités. Dans un mode de réalisation particulièrement simple et selon l'exemple de base de données ci-dessus, il pourrait se limiter à sélectionner, parmi les 150 objets étalons dont les paramètres spectraux de référence sont enregistrés dans la base de données 26, celui qui correspond le mieux aux mesures effectuées sur le matériau M. Mais de façon optionnelle et dans un mode de réalisation plus fin, il peut être procédé à une interpolation des paramètres spectraux de référence précités, permettant de sélectionner finalement des épaisseurs intermédiaires pour une plus grande précision de l'identification. Ainsi, l'univers des résultats possibles pour l'identification du matériau inconnu M est plus important que l'ensemble des 150 objets étalons.

Au cours d'une première étape de mesure 200 du procédé d'identification, un matériau à identifier M est placé entre l'émetteur 10 et le détecteur 12. Comme pour les étapes 100 et 102, au moins un spectre est obtenu en sortie du convertisseur 18, sous la forme d'un histogramme indiquant un nombre de photons reçus à chaque énergie, dans la gamme d'énergies discrètes prédéterminée. Si l'application visée impose une mesure rapide, un seul spectre est acquis. Sinon, plusieurs spectres peuvent être acquis et moyennés pour réduire l'impact du bruit photonique sur la mesure.

Là encore, il peut être imposé une valeur seuil en termes de nombre de photons reçus à chaque énergie, par exemple 5, en deçà de laquelle les valeurs du spectre mesuré ne sont pas prises en compte. Le spectre mesuré est donc défini dans au moins une bande d'énergies dans laquelle ses valeurs sont supérieures ou égales à la valeur seuil. Lors d'une étape 202, cette bande d'énergies est comparée à la bande d'émission $B_0$ et leur intersection est alors déterminée, définissant ainsi une bande de mesure Bm.

A chaque valeur d'énergie discrète de cette bande de mesure Bm, c'est-à-dire pour chacun de ses canaux, un coefficient spectral peut être déterminé (étape 204) selon l'équation (1) (coefficient de transmission) ou l'équation (2) (coefficient d'atténuation) pour l'obtention d'une fonction de comparaison liée au matériau à identifier M.

On notera que l'acquisition d'un spectre est réalisée par le détecteur 12, par exemple sur commande du calculateur 20, alors que les calculs d'un spectre moyen éventuel, le cas échéant, de la bande de mesure Bm et des coefficients spectraux déterminés à l'étape 204 sont effectués grâce à une exécution du programme 30 par le processeur 22.

Pour la suite de l'identification du matériau inconnu M, il convient de confronter les coefficients spectraux déterminés à l'étape 204 avec les paramètres spectraux de référence stockés dans la base de données 26. Cette confrontation est, comme déjà mentionné précédemment, soit une comparaison si les coefficients déterminés à l'étape 204 et les paramètres spectraux de référence sont de natures comparables, soit par exemple une maximisation de densité de probabilité ou de probabilités calculées par inférence bayésienne si les paramètres spectraux de référence sont des paramètres statistiques plus complexes. Plus généralement, cette confrontation comporte l'optimisation d'une fonction de coût (distance, densité de probabilité, probabilité, ...) paramétrée, pour chaque objet étalon Mref, à l'aide des paramètres spectraux de référence correspondants et à laquelle sont appliquées les valeurs des coefficients spectraux déterminés à l'étape 204.

Selon un mode de réalisation préféré illustré sur la figure 3, adapté notamment lorsque les objets étalons sont de plusieurs matériaux différents et de plusieurs épaisseurs différentes pour chaque matériau :

- dans une première phase PH1 incluant des étapes $206_i$ à $218_i$, exécutées indépendamment pour chaque matériau de référence, une épaisseur optimale (dans le sens d'une optimisation de la fonction de coût) est déterminée pour chaque matériau de référence, selon un procédé en au moins deux étapes incluant chacune une confrontation des coefficients spectraux déterminés à l'étape 204 avec une partie des paramètres spectraux de référence,
- dans une deuxième phase PH2 incluant des étapes 220 et 222, une épaisseur optimale, parmi l'ensemble des épaisseurs optimales déterminées dans la première phase, est sélectionnée sur la base d'une optimisation d'un critère évalué dans au moins une bande d'énergies commune à l'ensemble des bandes d'énergies dans lesquelles les épaisseurs optimales ont été déterminées.

Dans la suite de la description de la première phase PH1, seules les étapes $206_1$ à $218_1$ sont détaillées, relatives à un premier matériau M1 pour lequel N1 objets étalons d'épaisseurs différentes sont identifiés dans la base de données 26. Les étapes $206_i$ à $218_i$, pour $2 \leq i \leq p$ sont exécutées de la même manière pour tout matériau Mi pour lequel Ni objets étalons d'épaisseurs différentes sont identifiés dans la base de données 26.

Au cours d'une première étape $206_1$ de la première phase s'appliquant aux N1 objets étalons en matériau M1 de la base de données 26, réalisée sur exécution du programme 32, la bande de mesure Bm est comparée à chacune des bandes de référence $B_{1,1}$, ... $B_{1,N1}$ associées à ces N1 objets étalons. Une bande d'énergies $NE_{1,i}$ commune à toutes ces bandes de référence et à la bande de mesure, plus précisément l'intersection des bandes d'énergies $B_{1,1}$, ... $B_{1,N1}$ et Bm, est déterminée à partir de ces comparaisons.

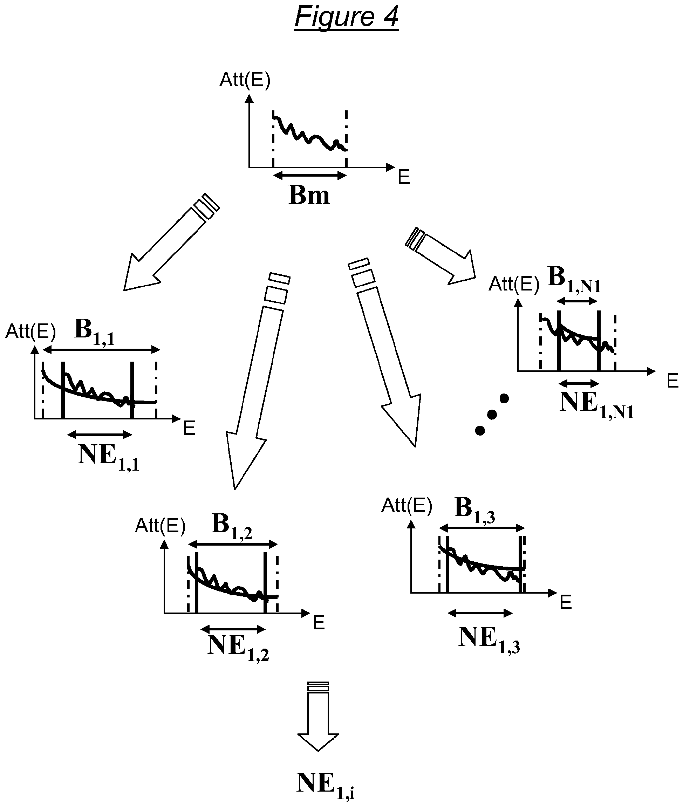

Comme plus précisément illustré sur la figure 4, cette détermination de la bande d'énergies commune $NE_{1,i}$ peut se faire en deux étapes. Une première étape consiste à comparer successivement la bande de mesure Bm à chacune des bandes de référence $B_{1,1}$, ... $B_{1,N1}$ pour en déterminer les intersections respectives $NE_{1,1}$, ... $NE_{1,N1}$. Puis ces intersections respectives sont comparées entre elles pour que celle qui présente le moins de canaux (i.e. le moins d'énergies discrètes) $NE_{1,i}$ soit sélectionnée en tant que bande d'énergies commune à toutes les bandes de référence $B_{1,1}$, ... $B_{1,N1}$ et à la bande de mesure Bm.

Lors d'une étape $208_1$ suivante de la première phase PH1, réalisée sur exécution du programme 32, n objets étalons en matériau M1 sont sélectionnés parmi les N1 de la base de données 26 en tant que candidats possibles pour le matériau inconnu M. Cette sélection se fait par confrontation des coefficients spectraux déterminés à l'étape 204 avec les paramètres spectraux de référence des N1 objets étalons en matériau M1 dans la bande d'énergies commune $NE_{1,i}$. Les n objets étalons optimisant la fonction de coût précédemment mentionnée et définie dans cette bande d'énergie commune $NE_{1,i}$ sont retenus. En pratique, n est compris entre 2 et N1-1. Dans l'exemple illustré sur la figure 3, il est plus précisément compris entre 3 et N1-1.

Ensuite, lors d'une étape $210_1$ suivante de la première phase PH1, réalisée sur une nouvelle itération du programme 32 et s'appliquant aux n candidats possibles sélectionnés lors de l'étape précédente, la bande de mesure Bm est comparée à chacune des bandes de référence associées à ces n candidats possibles. Une bande d'énergies commune à ces n bandes de référence et à la bande de mesure Bm est déterminée à partir de ces comparaisons. Puisque le nombre de bandes d'énergies dont l'intersection est recherchée (n+1) est inférieur à celui (N1+1) de l'étape $206_1$, nécessairement la bande d'énergie commune déterminée à cette étape inclut la bande d'énergie commune déterminée à l'étape $206_1$.

Comme l'étape $206_1$, cette étape est suivie d'une nouvelle étape de sélection analogue à l'étape $208_1$. Lors de cette nouvelle sélection, n' objets étalons en matériau M1 sont sélectionnés parmi les n candidats possibles en tant que nouveaux candidats possibles pour le matériau inconnu M. Cette sélection se fait par confrontation des coefficients spectraux déterminés à l'étape 204 avec les paramètres spectraux de référence des n candidats possibles dans la bande d'énergies commune déterminée à l'étape $210_1$. Les n' objets étalons, parmi les n candidats possibles, optimisant la fonction de coût précédemment mentionnée et définie dans cette nouvelle bande d'énergie commune sont retenus. En pratique n' est au plus égal à n-1.

Le programme 32 est itéré autant de fois que souhaité, possible et nécessaire pour finalement parvenir à une étape $212_1$ de sélection de deux objets étalons en matériau M1 parmi les derniers candidats possibles sélectionnés à l'étape de sélection précédente. Lors de cette étape de sélection $212_1$, deux objets étalons en matériau M1 sont sélectionnés en tant que nouveaux candidats possibles pour le matériau inconnu M. Cette sélection se fait par confrontation des coefficients spectraux déterminés à l'étape 204 avec les paramètres spectraux de référence des derniers candidats possibles retenus à l'étape de sélection précédente dans la bande d'énergies commune à la bande de mesure et aux bandes d'énergies de ces derniers candidats possibles. Les deux objets étalons, parmi les derniers candidats possibles, optimisant la fonction de coût précédemment mentionnée et définie dans cette nouvelle bande d'énergie commune sont retenus. On notera que l'étape $212_1$ est l'étape qui suit nécessairement directement l'étape $210_1$ si n=3. On notera également que la bande d'énergie commune dans laquelle se fait cette dernière étape de sélection inclut la bande d'énergie commune précédemment déterminée, qui elle-même inclut la bande d'énergie commune déterminée avant, et ainsi de suite. De la sorte, il est clair que plus la sélection se précise, plus la bande d'énergie commune dans laquelle cette sélection se fait est large et plus la sélection devient fiable.

En variante, dans un mode de réalisation particulièrement simple, l'étape de sélection $212_1$ pourrait consister en une sélection d'un unique objet étalon parmi les derniers candidats possibles, ce qui mettrait fin à la première phase PH1 du procédé d'identification pour le matériau M1.

Mais de façon optionnelle et dans un mode de réalisation plus fin, l'étape de sélection $212_1$ sélectionne deux objets étalons en matériau M1 parmi les derniers candidats possibles pour qu'elle puisse être suivie d'une interpolation entre ces deux objets étalons et d'une dernière sélection plus fine. On notera que, puisque de façon réaliste une seule épaisseur de matériau M1 doit optimiser la fonction de coût mentionnée précédemment, les deux objets étalons en matériau M1 sélectionnés sont d'épaisseurs successives dans la base de données 26. On notera enfin par ailleurs que dans le cas particulier (non illustré) où n serait égal à 2 à l'étape $208_1$, les itérations $210_1$, ..., $212_1$ seraient supprimées et c'est l'étape $208_1$ elle-même qui serait suivie d'une interpolation entre ces deux objets étalons et d'une dernière sélection plus fine.

Au cours d'une étape $214_1$ précédant l'interpolation $216_1$ et réalisée sur exécution du programme 34, la bande de mesure Bm est comparée à chacune des bandes de référence associées aux deux objets étalons sélectionnés parmi les derniers candidats possibles. Une bande d'énergies commune à ces deux bandes de référence et à la bande de mesure Bm, plus précisément l'intersection de ces trois bandes d'énergies, est déterminée à partir de ces comparaisons.

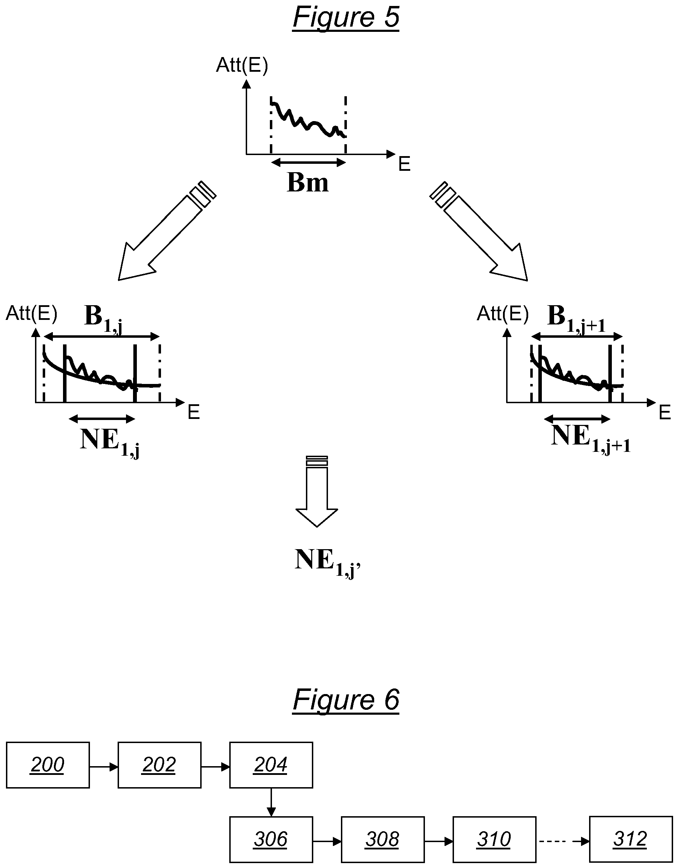

Comme plus précisément illustré sur la figure 5, cette détermination de la bande d'énergies commune peut se faire en deux étapes. Une première étape consiste à comparer successivement la bande de mesure Bm à chacune des bandes de référence $B_{1,j}$ et $B_{1,j+1}$ des deux objets étalons d'épaisseurs successives sélectionnés pour en déterminer les intersections respectives $NE_{1,j}$ et $NE_{1,j+1}$. Puis ces intersections respectives sont comparées entre elles pour que celle qui présente le moins de canaux (i.e. le moins d'énergies discrètes) $NE_{1,j'}$ soit sélectionnée en tant que bande d'énergies commune aux bandes de référence $B_{1,j}$ et $B_{1,j+1}$ et à la bande de mesure Bm.

L'interpolation $216_1$ consiste à appliquer une interpolation des paramètres spectraux de référence des deux objets étalons d'épaisseurs successives sélectionnés dans la bande d'énergies commune $NE_{1,j'}$. Cette interpolation se fait indépendamment dans chaque canal, c'est-à-dire dans l'exemple considéré pour chaque énergie discrète de la bande d'énergies commune $NE_{1,j'}$. Il s'agit en fait d'estimer des paramètres spectraux de référence supplémentaires supposés être relatifs à des objets étalons supplémentaires virtuels d'épaisseurs comprises entre les deux épaisseurs successives des deux objets étalons sélectionnés. Une façon très simple et bien connue de procéder à une telle interpolation est de choisir un pas de discrétisation en épaisseur, par exemple $1/N^e$ de la différence entre les deux épaisseurs successives des deux objets étalons sélectionnés (N=20 par exemple), puis d'interpoler linéairement chacun des paramètres spectraux de référence dans chaque canal par application d'une combinaison linéaire barycentrique des paramètres correspondants des deux objets étalons sélectionnés. On obtient ainsi N+1 objets étalons en matériau M1 de N+1 épaisseurs successives différentes, plus précisément les deux objets étalons sélectionnés et les N-1 objets étalons supplémentaires virtuels interpolés.

Cette étape d'interpolation $216_1$ est suivie d'une étape $218_1$ de sélection d'un unique objet étalon en matériau M1 parmi les N+1 objets étalons possibles. Cette sélection se fait par confrontation des coefficients spectraux déterminés à l'étape 204 avec les paramètres spectraux de référence de ces N+1 objets étalons possibles dans la bande d'énergies commune $NE_{1,j'}$ déterminée à l'étape $214_1$. L'objet étalon, pouvant notamment être virtuel, parmi les N+1 objets possibles, optimisant la fonction de coût précédemment mentionnée et définie dans la bande d'énergie commune $NE_{1,j'}$ est retenu. Cette étape $218_1$ met fin à la première phase PH1 du procédé d'identification par la sélection d'une épaisseur optimale E1 pour le matériau de référence M1.

Comme indiqué précédemment, on procède de même pour chaque matériau de référence Mi, $2 \leq i \leq p$, lors d'étapes similaires $206_i$ à $218_i$ exécutées indépendamment pour chaque matériau de référence. On obtient ainsi, en fin de première phase PH1, une sélection de p épaisseurs E1, ..., Ep, optimales respectivement pour les p matériaux de référence M1, ..., Mp, c'est-à-dire ayant optimisé des fonctions de coût aux étapes $218_1$, ..., $218_p$ dans respectivement p bandes d'énergies communes $NE_{1,j'}$, ... , $NE_{p,j'}$ déterminées respectivement aux étapes $214_1$, ..., $214_p$.

La deuxième phase PH2, réalisée par exécution du programme 34, comporte une étape 220 de détermination d'une bande d'énergies commune aux p bandes d'énergies communes $NE_{1,j'}$, ... , $NE_{p,j'}$ déterminées respectivement aux étapes $214_1$, ..., $214_p$. Plus précisément, cette étape 220 détermine l'intersection des p bandes d'énergies communes $NE_{1,j'}$, ... , $NE_{p,j'}$. Cette intersection est notée NE.

Enfin, la deuxième phase PH2 comporte une étape 222 de sélection finale d'une unique épaisseur optimale associée à un matériau de référence parmi les p épaisseurs optimales E1, ..., Ep. Cette sélection se fait par confrontation des coefficients spectraux déterminés à l'étape 204 avec les paramètres spectraux de référence de ces p objets étalons, réels ou virtuels, correspondants aux p épaisseurs optimales E1, ..., Ep dans la bande d'énergies commune NE. Eventuellement, si les calculs d'optimisations de fonctions de coût sont séparables d'un canal à l'autre dans la bande d'énergies commune NE, les résultats des étapes $218_1$, ..., $218_p$ peuvent être exploités dans cette étape 222 pour limiter la complexité du calcul. Le matériau de référence Mk et son épaisseur optimale Ek associée, parmi les p objets étalons, réels ou virtuels, correspondants aux p épaisseurs optimales E1, ..., Ep, optimisant la fonction de coût précédemment mentionnée et définie dans la bande d'énergie commune NE, sont retenus.

Le matériau inconnu M est donc identifié comme étant en matériau Mk et d'épaisseur Ek.

Il apparaît clairement qu'un procédé d'identification de matériau tel que celui décrit précédemment permet d'affiner progressivement la sélection en augmentant dans le même temps la bande d'énergie commune aux paramètres spectraux de référence pris en compte et aux coefficients spectraux issus de la mesure. L'augmentation de la bande d'énergie commune au fil de la sélection permet d'en améliorer la fiabilité et d'obtenir finalement une identification plus précise.

De nombreuses autres variantes de réalisation de l'invention que celles envisagées précédemment peuvent également être imaginées en conservant cette propriété avantageuse.

Notamment, conformément à un autre mode de réalisation plus simple illustré sur la figure 6, il n'est pas toujours nécessaire de procéder en deux phases PH1 et PH2 telles que mentionnées dans l'exemple illustré sur la figure 3. Les objets étalons pour lesquels on dispose de paramètres spectraux de référence ne sont pas nécessairement classés par nature, par épaisseur ou selon d'autres critères. Ils peuvent être considérés dans leur globalité comme N objets étalons auxquels le matériau à identifier M doit être comparé pour être identifié.

Selon cet autre mode de réalisation, l'étape 204 est suivie d'une étape 306 au cours de laquelle la bande de mesure Bm est comparée à toutes les bandes de référence des N objets étalons. Une bande d'énergies commune à toutes ces bandes de référence et à la bande de mesure, plus précisément l'intersection de toutes les bandes de référence et de la bande de mesure, est déterminée à partir de ces comparaisons.

L'étape 306 est suivie d'une étape 308 au cours de laquelle n candidats possibles sont sélectionnés parmi les N objets étalons. La valeur de n peut être choisie librement entre 2 et N-1. Cette sélection se fait par confrontation des coefficients spectraux déterminés à l'étape 204 avec les paramètres spectraux de référence des N objets étalons dans la bande d'énergies commune déterminée à l'étape 306.

Ensuite, lors d'une étape 310, la bande de mesure Bm est comparée à chacune des bandes de référence associées aux n candidats possibles. Une bande d'énergies commune à ces n bandes de référence et à la bande de mesure Bm est déterminée à partir de ces comparaisons. Puisque le nombre de bandes d'énergies dont l'intersection est recherchée est inférieur à celui de l'étape 306, nécessairement la bande d'énergie commune déterminée à cette étape inclut la bande d'énergie commune déterminée à l'étape 306.

Soit directement suite à l'étape 310, soit après plusieurs itérations de sélections successives (lors desquelles on réduit la sélection des candidats possibles à successivement n', n", ... avec N>n>n'>n" ...), on passe finalement à une étape 312 au cours de laquelle un unique objet étalon est sélectionné parmi les n (ou n', n", ... en cas de sélections successives) candidats possibles. Cette sélection se fait par confrontation des coefficients spectraux déterminés à l'étape 204 avec les paramètres spectraux de référence des n (ou n', n", ... en cas de sélections successives) candidats possibles dans la bande d'énergies commune à la bande de mesure et aux bandes de référence des candidats possibles. Le matériau inconnu M est alors identifié comme étant identique à l'objet étalon finalement sélectionné.

Il convient enfin de noter que chaque fois qu'il a été question de bande d'énergies, que ce soit la bande de mesure, les bandes de référence, ou les bandes d'énergies communes, une telle bande d'énergie n'est pas obligatoirement continue : elle peut être constituée de plusieurs bandes d'énergies disjointes.

**Confrontation des coefficients issus de la mesure avec les paramètres spectraux de référence**

On va maintenant préciser quelles formes peuvent prendre les confrontations entre coefficients spectraux issus de la mesure du matériau à identifier M et paramètres spectraux de référence dans une bande d'énergies donnée à NE canaux, selon différents modes de réalisation possibles. Ces confrontations sont celles qui ont été évoquées aux étapes $208_1$ ... $208_p$, $212_1$ ... $212_p$, $218_1$ ... $218_p$, 308 et 312.

Comme indiqué précédemment, selon un mode de réalisation possible, les paramètres spectraux de référence peuvent être des coefficients spectraux directement comparables aux coefficients spectraux mesurés pour le matériau inconnu M. En notant $CM = (CM_1, ..., CM_{NE})$ les coefficients spectraux issus de la mesure du matériau inconnu M et $CR = (CR_1, ..., CR_{NE})$ les coefficients spectraux de référence de l'un quelconque des objets étalons, la fonction de coût peut être définie comme une distance d à minimiser, prenant la forme suivante :

$$d(CM, CR) = \sqrt{\left(CM_1 - CR_1\right)^2 + ... + \left(CM_{NE} - CR_{NE}\right)^2}$$ , les valeurs $CM_i$ et $CR_i$, $1 \le i \le NE$, étant des scalaires.

Il peut également s'agir, plus simplement, d'une pseudo-distance :

$$d'(CM, CR) = \left(CM_1 - CR_1\right)^2 + ... + \left(CM_{NE} - CR_{NE}\right)^2 .$$

On remarque que les calculs $(CM_1 - CR_1)^2, ..., (CM_{NE} - CR_{NE})^2$ de ces fonctions de coût sont séparables d'un canal à l'autre (il suffit de les additionner entre eux) de sorte que lorsqu'ils sont mis en oeuvre dans les étapes $218_1$, ..., $218_p$ précitées, ils peuvent être judicieusement conservés en mémoire, par exemple sous forme vectorielle, pour être ensuite exploités à l'étape 222 puisque la bande d'énergies commune considérée lors de cette étape est l'intersection des bandes d'énergies communes considérées dans les étapes $214_1$, ..., $214_p$. Cela évite des calculs supplémentaires.

Comme indiqué précédemment, selon un autre mode de réalisation possible, les paramètres spectraux de référence peuvent être des paramètres statistiques, notamment des paramètres de densités de probabilités. A titre d'exemple non limitatif, sur la base d'un modèle de loi multinormale de dimension NE pouvant se simplifier, en prenant une hypothèse d'indépendance des NE canaux, en NE lois normales indépendantes opérant chacune dans l'un des canaux de la bande d'énergies donnée, on peut noter $\mu R = (\mu R_1, ..., \mu R_{NE})$ et $\sigma R = (\sigma R_1, ..., \sigma R_{NE})$ respectivement les paramètres spectraux de référence « moyenne » et « écart-type » de l'un quelconque des objets étalons. La fonction de coût peut alors être définie comme une densité de probabilité à maximiser, prenant la forme suivante :

$$f_{\mu R, \sigma R}(CM) = \prod_{i=1}^{NE} \frac{1}{\sqrt{2\pi} \sigma R_i} . \exp\left[ -\frac{1}{2}\left( \frac{CM_i - \mu R_i}{\sigma R_i} \right)^2 \right] .$$

On remarque la aussi que les calculs $\frac{1}{\sqrt{2\pi}\sigma R_1}.exp\left[-\frac{1}{2}\left(\frac{CM_1-\mu R_1}{\sigma R_1}\right)^2\right]$, ...,

$\frac{1}{\sqrt{2\pi}\sigma R_{NE}}.exp\left[-\frac{1}{2}\left(\frac{CM_{NE}-\mu R_{NE}}{\sigma R_{NE}}\right)^2\right]$ de cette fonction de coût sont séparables d'un canal à l'autre (il suffit de les multiplier entre eux) de sorte que lorsqu'ils sont mis en oeuvre dans les étapes $218_1$, ..., $218_p$ précitées, ils peuvent être judicieusement conservés en mémoire, par exemple sous forme vectorielle, pour être ensuite exploités à l'étape 222 puisque la bande d'énergies commune considérée lors de cette étape est l'intersection des bandes d'énergies communes considérées dans les étapes $214_1$, ..., $214_p$. Cela évite des calculs supplémentaires.

Bien sûr, il est également possible de prendre un modèle de loi multinormale plus complexe, c'est-à-dire ne supposant pas que les canaux soient indépendants entre eux. Dans ce cas, La fonction de coût prend une forme plus complexe incluant la prise en compte d'une forme plus générale de la matrice de covariance. Cette forme plus complexe et les calculs de covariance étant bien connus, ils ne seront pas détaillés ici. Il est possible aussi de choisir d'autres modèles statistiques que des lois normales.

Dans cet autre mode de réalisation, la fonction de coût peut également prendre une autre forme qu'une densité de probabilité à maximiser : par exemple une probabilité conditionnelle directement calculée et à maximiser également. Plus précisément, cette probabilité conditionnelle est la probabilité a posteriori conditionnelle, notée $p_{CM}[(\mu R, \sigma R)]$, que le matériau à identifier M soit celui correspondant aux paramètres spectraux de référence $(\mu R, \sigma R)$ connaissant la valeur CM des coefficients spectraux issus de la mesure.

Selon le théorème de Bayes, cette probabilité peut se décomposer comme suit :

$$p_{CM}\left[(\mu R,\sigma R)\right] = p_{\mu R,\sigma R}(CM)\cdot\frac{p\left[(\mu R,\sigma R)\right]}{p(CM)}, \qquad (3)$$

où $p_{\mu R, \sigma R}(CM)$ est la probabilité a posteriori conditionnelle d'obtenir une valeur CM de coefficients spectraux issus de la mesure pour l'objet étalon de paramètres spectraux de référence $(\mu R, \sigma R)$, $p[(\mu R, \sigma R)]$ est la probabilité a priori d'être en présence de l'objet étalon de paramètres spectraux de référence $(\mu R, \sigma R)$ et $p(CM)$ est la probabilité a priori de la valeur CM de coefficients spectraux issus de la mesure.

A titre d'exemple non limitatif, comme précédemment, sur la base d'un modèle de loi multinormale de dimension NE pouvant se simplifier, en prenant une hypothèse d'indépendance des NE canaux, en NE lois normales indépendantes opérant chacune dans l'un des canaux de la bande d'énergies donnée, on peut noter $\mu R = (\mu R_1, ..., \mu R_{NE})$ et $\sigma R = (\sigma R_1, ..., \sigma R_{NE})$ respectivement les paramètres spectraux de référence « moyenne » et « écart-type » de l'un quelconque des objets étalons. Par ailleurs, en notant p le nombre de matériaux de référence et Nk le nombre d'épaisseurs de référence pour le k-ième matériau de référence (l'univers des épaisseurs de référence comprend alors non seulement celles pour lesquelles un calibrage a été effectué, mais en outre celles pour lesquelles les paramètres spectraux de référence ont été obtenus par interpolation), il est possible d'expliciter chacun des termes de droite de l'équation (3).

$$\text{Ainsi, } p\left[(\mu R,\sigma R)\right] = \frac{1}{p}\cdot\frac{1}{Nk}, \qquad (4)$$

où k désigne le matériau de l'objet étalon de paramètres spectraux de référence $(\mu R, \sigma R)$.

Ainsi également, $p_{\mu R,\sigma R}(CM) = \prod_{i=1}^{NE} p_{\mu R_i,\sigma R_i}(CM_i)$ par indépendance des canaux.

Par ailleurs, pour tout i, en encadrant au plus près la valeur de $CM_i$ entre une borne inférieure $Inf(CM_i)$ et une borne supérieure $Sup(CM_i)$, on peut écrire :
$p_{\mu R_i,\sigma R_i}(CM_i) = p_{\mu R_i,\sigma R_i}(Inf(CM_i) < CM_i < Sup(CM_i))$, soit, sous une loi normale :

$$p_{\mu R_i,\sigma R_i}(CM_i) = \int_{Inf(CM_i)}^{Sup(CM_i)}\frac{1}{\sqrt{2\pi}\sigma R_i}.exp\left[-\frac{1}{2}\left(\frac{x-\mu R_i}{\sigma R_i}\right)^2\right]dx.$$

Il en résulte l'expression :

$$p_{\mu R,\sigma R}(CM) = \prod_{i=1}^{NE} \int_{Inf(CM_i)}^{Sup(CM_i)} \frac{1}{\sqrt{2\pi}\sigma R_i} \cdot \exp\left[-\frac{1}{2}\left(\frac{x-\mu R_i}{\sigma R_i}\right)^2\right] dx \,. \tag{5}$$

Pour tout i, puisque la valeur de $CM_i$ dépend de la fonction de comparaison choisie, alors les valeurs de $Inf(CM_i)$ et $Sup(CM_i)$ en sont également dépendantes.

Ainsi par exemple, si la fonction de comparaison prend la forme d'une fonction de transmission, la valeur de $CM_i$ est $CM_i = \dfrac{I(i)}{I_0(i)}$, où $I(i)$ et $I_0(i)$ désignent respectivement la mesure du nombre de photons transmis par le matériau à identifier et celle du nombre de photons incidents dans le i-ème canal.

Or on peut encadrer le nombre $I(i)$ au plus près par :

$$I(i)-1 < I(i) < I(i)+1 \text{, soit } \frac{I(i)-1}{I_0(i)} < \frac{I(i)}{I_0(i)} < \frac{I(i)+1}{I_0(i)} \,.$$

D'où, dans le cas d'une fonction de transmission: $Inf(CM_i) = \dfrac{I(i)-1}{I_0(i)}$ et $Sup(CM_i) = \dfrac{I(i)+1}{I_0(i)}$.

Si la fonction de comparaison prend la forme d'une fonction d'atténuation, par un calcul similaire on obtient

$$Inf(CM_i) = -\ln\left(\frac{I(i)+1}{I_0(i)}\right) \text{ et } Sup(CM_i) = -\ln\left(\frac{I(i)-1}{I_0(i)}\right).$$

Enfin, le dernier terme de l'équation (3) s'explicite de la façon suivante, par un développement à l'aide de la loi des probabilités totales sur tous les objets étalons dans tout l'univers des épaisseurs de référence :

$$p(CM) = \frac{1}{p} \cdot \sum_l \frac{1}{Nl} \cdot \sum_{j=1}^{Nl} p_{\mu R(l,j),\sigma R(l,j)}(CM) = \frac{1}{p} \cdot \sum_l \frac{1}{Nl} \cdot \sum_{j=1}^{Nl} \prod_{i=1}^{NE} p_{\mu R(l,j)_i,\sigma R(l,j)_i}(CM_i) \,.$$

Sous une loi multinormale, il en résulte l'expression suivante :

$$p(CM) = \frac{1}{p} \cdot \sum_l \frac{1}{Nl} \cdot \sum_{j=1}^{Nl} \prod_{i=1}^{NE} \int_{Inf(CM_i)}^{Sup(CM_i)} \frac{1}{\sqrt{2\pi}\sigma R(l,j)_i} \cdot \exp\left[-\frac{1}{2}\left(\frac{x-\mu R(l,j)_i}{\sigma R(l,j)_i}\right)^2\right] dx \,. \tag{6}$$

En reportant les équations (4), (5) et (6) dans l'équation (3), on obtient finalement l'expression (7) suivante :

$$p_{CM}[(\mu R,\sigma R)] = \frac{1}{Nk} \cdot \frac{\displaystyle\prod_{i=1}^{NE} \int_{Inf(CM_i)}^{Sup(CM_i)} \frac{1}{\sqrt{2\pi}\sigma R_i} \cdot \exp\left[-\frac{1}{2}\left(\frac{x-\mu R_i}{\sigma R_i}\right)^2\right] dx}{\displaystyle\sum_l \frac{1}{Nl} \cdot \sum_{j=1}^{Nl} \prod_{i=1}^{NE} \int_{Inf(CM_i)}^{Sup(CM_i)} \frac{1}{\sqrt{2\pi}\sigma R(l,j)_i} \cdot \exp\left[-\frac{1}{2}\left(\frac{x-\mu R(l,j)_i}{\sigma R(l,j)_i}\right)^2\right] dx} \,.$$

Cette expression peut se simplifier. En effet, puisque l'on cherche, dans l'univers des objets étalons possibles (y compris pour les épaisseurs de référence interpolées), celui qui maximise la probabilité $p_{CM}[(\mu R,\sigma R)]$, il est suffisant de connaître cette dernière à une constante près. Or la probabilité a priori $p(CM)$ est indépendante des objets étalons, par conséquent elle constitue simplement un facteur de normalisation à 1 dans l'équation (3). Il n'est donc pas utile de la calculer, ce qui simplifie sensiblement l'expression (7).

Par ailleurs, on peut procéder à un changement de variable dans l'équation (5) de manière à l'exprimer à l'aide de la fonction spéciale d'erreur de Gauss *erf* qui, elle, est bien connue. Plus précisément, on peut remarquer que :

$$\int_{Inf(CM_i)}^{Sup(CM_i)} \frac{1}{\sqrt{2\pi}\sigma R_i} \cdot \exp\left[-\frac{1}{2}\left(\frac{x-\mu R_i}{\sigma R_i}\right)^2\right] dx = \frac{1}{2}\cdot\left(1+erf\left(\frac{Sup(CM_i)-\mu R_i}{2\cdot\sigma R_i}\right)\right)$$
$$-\frac{1}{2}\cdot\left(1+erf\left(\frac{Inf(CM_i)-\mu R_i}{2\cdot\sigma R_i}\right)\right)$$

L'expression (7) se simplifie donc comme suit pour donner une expression facilement calculable de la fonction de coût :

$$p_{CM}\left[(\mu R, \sigma R)\right] \propto \frac{1}{Nk}\cdot\prod_{i=1}^{NE}\left[erf\left(\frac{Sup(CM_i)-\mu R_i}{2\cdot\sigma R_i}\right)-erf\left(\frac{Inf(CM_i)-\mu R_i}{2\cdot\sigma R_i}\right)\right].$$

Un avantage de cette fonction de coût est d'être normalisée (à une constante près). En outre, comme pour les deux fonctions de coût précédentes, ses calculs $\left[erf\left(\frac{Sup(CM_i)-\mu R_i}{2\cdot\sigma R_i}\right)-erf\left(\frac{Inf(CM_i)-\mu R_i}{2\cdot\sigma R_i}\right)\right]$ sont séparables d'un canal à l'autre (il suffit de les multiplier entre eux) de sorte que lorsqu'ils sont mis en oeuvre dans les étapes $218_1$, ..., $218_p$ précitées, ils peuvent être judicieusement conservés en mémoire, par exemple sous forme vectorielle, pour être ensuite exploités à l'étape 222 puisque la bande d'énergies commune considérée lors de cette étape est l'intersection des bandes d'énergies communes considérées dans les étapes $214_1$, ..., $214_p$. Cela évite des calculs supplémentaires.

D'autres fonctions de coût peuvent encore être utilisées sur la base des paramètres spectraux de référence précités.

Il convient de noter qu'un procédé d'identification d'un matériau selon l'invention est indépendant de la façon dont les confrontations entre coefficients spectraux issus de la mesure et paramètres spectraux de référence se font concrètement. Ainsi, les exemples de confrontations précités s'appliquent avantageusement à un procédé d'identification de matériau selon l'invention, mais d'autres méthodes non décrites pourraient tout autant s'appliquer à ce procédé. Inversement, les exemples de confrontations précités peuvent s'appliquer à d'autres procédés d'identification de matériaux que ceux envisagés précédemment.

On notera enfin que l'invention n'est pas limitée aux modes de réalisation décrits précédemment. Il apparaîtra en effet à l'homme de l'art que diverses modifications peuvent être apportées aux modes de réalisation décrits ci-dessus, à la lumière de l'enseignement qui vient de lui être divulgué. Dans les revendications qui suivent, les termes utilisés ne doivent pas être interprétés comme limitant les revendications aux modes de réalisation exposés dans la présente description, mais doivent être interprétés pour y inclure tous les équivalents que les revendications visent à couvrir du fait de leur formulation et dont la prévision est à la portée de l'homme de l'art en appliquant ses connaissances générales à la mise en oeuvre de l'enseignement qui vient de lui être divulgué.

**Revendications**

1. Procédé d'identification d'un matériau (M) par analyse spectrale de rayonnements électromagnétiques aptes à traverser ce matériau, comportant les étapes suivantes :

   - mesure (200) d'un spectre d'un rayonnement électromagnétique transmis à travers le matériau à identifier (M),
   - détermination (202, 204) d'au moins une bande d'énergies (Bm), dite bande de mesure, et de coefficients spectraux d'une fonction de comparaison dans cette bande de mesure, à partir du spectre mesuré,
   - estimation (PH1, PH2 ; 306, 308, 310, 312), à l'aide des coefficients spectraux déterminés, de la nature et/ou de l'épaisseur du matériau à identifier (M) sur la base d'un ensemble de paramètres spectraux de référence relatifs à des matériaux et/ou épaisseurs de référence, ces paramètres spectraux de référence étant définis pour chaque matériau et/ou épaisseur de référence dans au moins une bande d'énergies dite bande de référence propre à chaque matériau et/ou épaisseur de référence,

**caractérisé en ce que** l'estimation comporte au moins les deux étapes suivantes :

- sélection préalable ($208_1$, ..., $208_p$ ; 308) d'une pluralité de matériaux et/ou épaisseurs de référence, dits candidats possibles, à partir d'une confrontation des coefficients spectraux déterminés avec au moins une partie des paramètres spectraux de référence dans une première bande d'énergies commune correspondant à l'intersection d'au moins une partie des bandes de référence et de la bande de mesure,
- détermination ($210_1$, ..., $210_p$ ; 310) d'au moins une nouvelle bande d'énergies commune correspondant à l'intersection des bandes de référence d'au moins une partie des candidats possibles et de la bande de mesure,
- estimation (222 ; 312) de la nature et/ou de l'épaisseur du matériau à identifier (M) à partir d'une confrontation des coefficients spectraux déterminés avec les paramètres spectraux de ladite au moins une partie des candidats possibles, dans ladite nouvelle bande d'énergies commune.

2. Procédé d'identification d'un matériau (M1) selon la revendication 1, dans lequel la sélection préalable ($208_1$, ..., $208_p$ ; 308) des candidats possibles se fait :

- soit dans au moins une bande d'énergies commune à toutes les bandes de référence et à la bande de mesure (Bm),
- soit dans au moins une bande d'énergies ($NE_{1,i}$) commune à une partie prédéterminée des bandes de référence ($B_{1,1}$, ..., $B_{1,N1}$) et à la bande de mesure (Bm).

3. Procédé d'identification d'un matériau (M) selon la revendication 1 ou 2, dans lequel les matériaux et/ou épaisseurs de référence comportent une pluralité de matériaux de référence à une pluralité d'épaisseurs de référence chacun.

4. Procédé d'identification d'un matériau (M) selon la revendication 3, dans lequel l'estimation comporte les étapes suivantes, exécutées pour chaque matériau de référence :

- sélection ($208_1$, ..., $208_p$ ; 308) d'une pluralité d'épaisseurs de référence pour ce matériau de référence, dites candidates possibles, par confrontation des coefficients spectraux déterminés avec les paramètres spectraux de référence dans au moins une bande d'énergies ($NE_{1,i}$) commune aux bandes de référence de chaque épaisseur de référence ($B_{1,1}$, ..., $B_{1,N1}$) pour ce matériau de référence et à la bande de mesure (Bm),
- sélection ($212_1$, ..., $212_p$) de deux épaisseurs de référence consécutives pour ce matériau de référence par confrontation des coefficients spectraux déterminés avec les paramètres spectraux d'au moins une partie des candidats possibles, dans au moins une bande d'énergies commune aux bandes de référence de ladite au moins une partie des candidats possibles et à la bande de mesure (Bm),
- estimation ($218_1$, ..., $218_p$) d'une épaisseur optimale pour ce matériau de référence par confrontation des coefficients spectraux déterminés avec des paramètres spectraux interpolés à partir des paramètres spectraux relatifs aux deux épaisseurs de référence consécutives sélectionnées, dans au moins une bande d'énergies ($NE_{1,j'}$) commune aux bandes de référence des deux épaisseurs de référence consécutives sélectionnées ($B_{1,j}$, $B_{1,j+1}$) et à la bande de mesure (Bm).

5. Procédé d'identification d'un matériau (M) selon la revendication 4, dans lequel l'estimation de la nature et/ou de l'épaisseur du matériau à identifier comporte la sélection (222) d'une épaisseur optimale, parmi l'ensemble des épaisseurs optimales associées chacune à un matériau de référence, sur la base d'une optimisation d'un critère évalué dans au moins une bande d'énergies commune à l'ensemble des bandes d'énergies dans lesquelles les épaisseurs optimales ont été estimées.

6. Procédé d'identification d'un matériau (M) selon l'une quelconque des revendications 1 à 5, dans lequel les paramètres spectraux de référence relatifs à des matériaux et/ou épaisseurs de référence sont des paramètres statistiques de densités de probabilité, chaque matériau et/ou épaisseur de référence étant ainsi associé à une densité de probabilité permettant le calcul d'une probabilité de mesurer des coefficients spectraux en présence de ce matériau et/ou épaisseur de référence dans sa bande de référence.

7. Procédé d'identification d'un matériau (M) selon la revendication 6, dans lequel la confrontation des coefficients spectraux déterminés avec les paramètres spectraux de référence relatifs à un matériau et/ou une épaisseur de référence comporte l'estimation d'une valeur que prend la densité de probabilité de ce matériau et/ou cette épaisseur de référence lorsque lui sont appliqués les coefficients spectraux déterminés.

8. Procédé d'identification d'un matériau (M) selon la revendication 6, dans lequel la confrontation des coefficients spectraux déterminés avec les paramètres spectraux de référence relatifs à un matériau et/ou épaisseur de référence comporte l'estimation d'une probabilité a posteriori conditionnelle d'être en présence de ce matériau et/ou épaisseur de référence connaissant les coefficients spectraux déterminés, sur la base du produit d'une probabilité a posteriori conditionnelle de mesurer les coefficients spectraux déterminés en présence de ce matériau et/ou épaisseur de référence et d'une probabilité a priori d'être en présence de ce matériau et/ou épaisseur de référence, par application du théorème de Bayes.

9. Dispositif d'identification d'un matériau (M) par analyse spectrale de rayonnements électromagnétiques aptes à traverser ce matériau, comportant :

- un émetteur (10) de rayonnement électromagnétique,
- un dispositif (12) de mesure d'un spectre d'un rayonnement électromagnétique transmis à travers le matériau à identifier,
- un calculateur (20) programmé (28, 30, 32, 34) pour :

• déterminer (202, 204) au moins une bande d'énergies (Bm), dite bande de mesure, et des coefficients spectraux d'une fonction de comparaison dans cette bande de mesure, à partir du spectre mesuré, et
• estimer (PH1, PH2 ; 306, 308, 310, 312), à l'aide des coefficients spectraux déterminés, la nature et/ou l'épaisseur du matériau à identifier (M) sur la base d'un ensemble de paramètres spectraux de référence relatifs à des matériaux et/ou épaisseurs de référence, ces paramètres spectraux de référence étant définis pour chaque matériau et/ou épaisseur de référence dans au moins une bande d'énergies dite bande de référence propre à chaque matériau et/ou épaisseur de référence,

**caractérisé en ce que** le calculateur (20) est plus précisément programmé pour :

- sélectionner préalablement ($208_1$, ..., $208_p$ ; 308) une pluralité de matériaux et/ou épaisseurs de référence, dits candidats possibles, à partir d'une confrontation des coefficients spectraux déterminés avec au moins une partie des paramètres spectraux de référence dans une première bande d'énergies commune correspondant à l'intersection d'au moins une partie des bandes de référence et de la bande de mesure,
- déterminer ($210_1$, ..., $210_p$ ; 310) au moins une nouvelle bande d'énergies commune correspondant à l'intersection des bandes de référence d'au moins une partie des candidats possibles et de la bande de mesure,
- estimer (222 ; 312) la nature et/ou l'épaisseur du matériau à identifier (M) à partir d'une confrontation des coefficients spectraux déterminés avec les paramètres spectraux de ladite au moins une partie des candidats possibles, dans ladite nouvelle bande d'énergies commune.

10. Programme d'ordinateur téléchargeable depuis un réseau de communication et/ou enregistré sur un support lisible par ordinateur et/ou exécutable par un processeur, **caractérisé en ce qu'**il comprend des instructions pour l'exécution des étapes d'un procédé d'identification d'un matériau (M) selon l'une quelconque des revendications 1 à 8, lorsque ledit programme est exécuté sur un ordinateur.

**Patentansprüche**

1. Verfahren zum Identifizieren eines Materials (M) durch Spektralanalyse von elektromagnetischen Strahlungen, die in der Lage sind, dieses Material zu durchdringen, umfassend die folgenden Schritte:

- Messen (200) eines Spektrums einer elektromagnetischen Strahlung, die durch das zu identifizierende Material (M) hindurchgeleitet wird,
- Bestimmen (202, 204) von mindestens einem als Messband bezeichneten Energieband (Bm) und von Spektralkoeffizienten einer Vergleichsfunktion in diesem Messband auf Grundlage des gemessenen Spektrums,
- Schätzen (PH1, PH2; 306, 308, 310, 312) der Art und/oder der Dicke des zu identifizierenden Materials (M) mithilfe der bestimmten Spektralkoeffizienten auf der Basis eines Satzes Referenzspektralparameter, die sich auf Referenzmaterialien und/oder Referenzdicken beziehen, wobei diese Referenzspektralparameter für jedes Referenzmaterial und/oder jede Referenzdicke in mindestens einem als Referenzband bezeichneten Energieband bestimmt werden, das jedem Referenzmaterial und/oder jeder Referenzdicke eigen ist,

**dadurch gekennzeichnet, dass** das Schätzen mindestens die folgenden zwei Schritte umfasst:

- vorheriges Auswählen ($208_1$, ..., $208_p$; 308) einer Vielzahl von als mögliche Kandidaten bezeichneten Referenzmaterialien und/oder Referenzdicken auf Grundlage einer Gegenüberstellung der bestimmten Spektralkoeffizienten mit mindestens einem Teil der Referenzspektralparameter in einem ersten gemeinsamen Energieband, das der Schnittmenge mindestens eines Teils der Referenzbänder und des Messbandes entspricht,

- Bestimmen ($210_1$, ..., $210_p$; 310) von mindestens einem neuen gemeinsamen Energieband, das der Schnittmenge der Referenzbänder mindestens eines Teils der möglichen Kandidaten und des Messbandes entspricht,

- Schätzen (222; 312) der Art und/oder der Dicke des zu identifizierenden Materials (M) auf Grundlage einer Gegenüberstellung der bestimmten Spektralkoeffizienten mit den Spektralparametern des mindestens einen Teils der möglichen Kandidaten im neuen gemeinsamen Energieband.

2. Verfahren zum Identifizieren eines Materials (M1) nach Anspruch 1, wobei das vorherige Auswählen ($208_1$, ..., $208_p$; 308) der möglichen Kandidaten erfolgt:

- entweder in mindestens einem Energieband, das allen den Referenzbändern und dem Messband (Bm) gemein ist,

- oder in mindestens einem Energieband ($NE_{1,i}$), das einem vorbestimmten Teil der Referenzbänder ($B_{1,1}$, ..., $B_{1,N1}$) und dem Messband (Bm) gemein ist.

3. Verfahren zum Identifizieren eines Materials (M) nach Anspruch 1 oder 2, wobei die Referenzmaterialien und/oder Referenzdicken eine Vielzahl von Referenzmaterialien, jedes in einer Vielzahl von Referenzdicken, umfassen.

4. Verfahren zum Identifizieren eines Materials (M) nach Anspruch 3, wobei das Schätzen die folgenden Schritte umfasst, die für jedes Referenzmaterial ausgeführt werden:

- Auswählen ($208_1$, ..., $208_p$; 308) einer Vielzahl von als mögliche Kandidaten bezeichneten Referenzdicken für dieses Referenzmaterial durch Gegenüberstellung der bestimmten Spektralkoeffizienten mit den Referenzspektralparametern in mindestens einem Energieband ($NE_{1,i}$), das den Referenzbändern jeder Referenzdicke ($B_{1,1}$, ..., $B_{1,N1}$) für dieses Referenzmaterial und dem Messband (Bm) gemein ist,

- Auswählen ($212_1$, ..., $212_p$) von zwei aufeinanderfolgenden Referenzdicken für dieses Referenzmaterial durch Gegenüberstellung der bestimmten Spektralkoeffizienten mit den Spektralparametern mindestens eines Teils der möglichen Kandidaten in mindestens einem Energieband, das den Referenzbändern des mindestens einen Teils der möglichen Kandidaten und dem Messband (Bm) gemein ist,

- Schätzen ($218_1$, ..., $218_p$) einer optimalen Dicke für dieses Referenzmaterial durch Gegenüberstellung der bestimmten Spektralkoeffizienten mit auf Grundlage der sich auf die zwei ausgewählten aufeinanderfolgenden Referenzdicken beziehenden Spektralparameter interpolierten Spektralparametern in mindestens einem Energieband ($NE_{1,j'}$), das den Referenzbändern der zwei ausgewählten aufeinanderfolgenden Referenzdicken ($B_{1,j}$, $B_{1,j+1}$) und dem Messband (Bm) gemein ist.

5. Verfahren zum Identifizieren eines Materials (M) nach Anspruch 4, wobei das Schätzen der Art und/oder der Dicke des zu identifizierenden Materials das Auswählen (222) einer optimalen Dicke aus dem Satz der optimalen Dicken, die jede mit einem Referenzmaterial assoziiert sind, auf der Basis einer Optimierung eines Kriteriums umfasst, das in mindestens einem Energieband bewertet wird, welches dem Satz der Energiebänder, in denen die optimalen Dicken geschätzt wurden, gemein ist.

6. Verfahren zum Identifizieren eines Materials (M) nach einem der Ansprüche 1 bis 5, wobei die Referenzspektralparameter, die sich auf Referenzmaterialien und/oder Referenzdicken beziehen, statistische Wahrscheinlichkeitsdichte-Parameter sind, wobei jedes Referenzmaterial und/oder jede Referenzdicke, das/die so mit einer Wahrscheinlichkeitsdichte assoziiert wird, die Berechnung einer Wahrscheinlichkeit, in Gegenwart dieses Referenzmaterials und/oder dieser Referenzdicke Spektralkoeffizienten in seinem/ihrem Referenzband zu messen, ermöglicht.

7. Verfahren zum Identifizieren eines Materials (M) nach Anspruch 6, wobei die Gegenüberstellung der bestimmten Spektralkoeffizienten mit den Referenzspektralparametern, die sich auf ein Referenzmaterial und/oder eine Referenzdicke beziehen, das Schätzen eines Wertes umfasst, den die Wahrscheinlichkeitsdichte dieses Referenzmaterials und/oder dieser Referenzdicke annimmt, wenn bestimmte Spektralkoeffizienten auf sie angewendet werden.

8. Verfahren zum Identifizieren eines Materials (M) nach Anspruch 6, wobei die Gegenüberstellung der bestimmten Spektralkoeffizienten mit den Referenzspektralparametern, die sich auf ein Referenzmaterial und/oder eine Referenzdicke beziehen, das Schätzen einer bedingten A-posteriori-Wahrscheinlichkeit, sich in Gegenwart dieses Referenzmaterials und/oder dieser Referenzdicke zu befinden, unter Kenntnis der bestimmten Spektralkoeffizienten auf der Basis des Produkts einer bedingten A-posteriori-Wahrscheinlichkeit, die bestimmten Spektralkoeffizienten in Gegenwart dieses Referenzmaterials und/oder dieser Referenzdicke zu messen, und einer A-priori-Wahrscheinlichkeit, sich in Gegenwart dieses Referenzmaterials und/oder dieser Referenzdicke zu befinden, durch Anwendung des Bayes-Theorems umfasst.

9. Vorrichtung zum Identifizieren eines Materials (M) durch Spektralanalyse von elektromagnetischen Strahlungen, die in der Lage sind, dieses Material zu durchdringen, umfassend:

- einen Sender (10) von elektromagnetischer Strahlung,
- eine Vorrichtung (12) zum Messen eines Spektrums einer elektromagnetischen Strahlung, die durch das zu identifizierende Material hindurchgeleitet wird,
- einen Rechner (20), der dafür programmiert (28, 30, 32, 34) ist:

-- mindestens ein als Messband bezeichnetes Energieband (Bm) und Spektralkoeffizienten einer Vergleichsfunktion in diesem Messband auf Grundlage des gemessenen Spektrums zu bestimmen (202, 204), und

-- mithilfe der bestimmten Spektralkoeffizienten die Art und/oder die Dicke des zu identifizierenden Materials (M) auf der Basis eines Satzes Referenzspektralparameter, die sich auf Referenzmaterialien und/oder Referenzdicken beziehen, zu schätzen (PH1, PH2; 306, 308, 310, 312), wobei diese Referenzspektralparameter für jedes Referenzmaterial und/oder jede Referenzdicke in mindestens einem als Referenzband bezeichneten Energieband bestimmt werden, das jedem Referenzmaterial und/oder jeder Referenzdicke eigen ist,

**dadurch gekennzeichnet, dass** der Rechner (20) genauer dafür programmiert ist:

- vorher eine Vielzahl von als mögliche Kandidaten bezeichneten Referenzmaterialien und/oder Referenzdicken auf Grundlage einer Gegenüberstellung der bestimmten Spektralkoeffizienten mit mindestens einem Teil der Referenzspektralparameter in einem ersten gemeinsamen Energieband, das der Schnittmenge mindestens eines Teils der Referenzbänder und des Messbandes entspricht, auszuwählen ($208_1$, ..., $208_p$; 308),
- mindestens ein neues gemeinsames Energieband, das der Schnittmenge der Referenzbänder mindestens eines Teils der möglichen Kandidaten und des Messbandes entspricht, zu bestimmen ($210_1$, ..., $210_p$; 310),
- die Art und/oder die Dicke des zu identifizierenden Materials (M) auf Grundlage einer Gegenüberstellung der bestimmten Spektralkoeffizienten mit den Spektralparametern des mindestens einen Teils der möglichen Kandidaten im neuen gemeinsamen Energieband zu schätzen (222; 312).

10. Computerprogramm, das aus einem Kommunikationsnetz heruntergeladen werden kann und/oder auf einem computerlesbaren Träger aufgezeichnet ist und/oder von einem Prozessor ausgeführt werden kann, **dadurch gekennzeichnet, dass** es Anweisungen für die Ausführung der Schritte eines Verfahrens zum Identifizieren eines Materials (M) nach einem der Ansprüche 1 bis 8 umfasst, wenn das Programm auf einem Computer ausgeführt wird.

## Claims

1. Method for identifying a material (M) by spectral analysis of electromagnetic radiation able to pass through this material, comprising the following steps:

- measuring (200) a spectrum of electromagnetic radiation emitted through the material to be identified (M),
- determining (202, 204) at least one energy band (Bm), referred to as a measurement band, and spectral coefficients of a comparison function in this measurement band, using the measured spectrum,
- estimating (PH1, PH2; 306, 308, 310, 312), using the determined spectral coefficients, the nature and/or the thickness of the material to be identified (M) on the basis of a set of reference spectral parameters relating to reference materials and/or thicknesses, these reference spectral parameters being defined for each reference material and/or thickness in at least one energy band referred to as a reference band specific to each reference material and/or thickness,

**characterized in that** the estimation comprises at least the two following steps:

- prior selecting (208$_1$, ..., 208$_p$; 308) of a plurality of reference materials and/or thicknesses, referred to as possible candidates, from a comparison of the spectral coefficients determined with at least one portion of the reference spectral parameters in a first common energy band that corresponds to the intersection of at least one portion of the reference bands and the measurement band,
- determining (210$_1$, ..., 210$_p$; 310) at least one new common energy band that corresponds to the intersection of the reference bands of at least one portion of the possible candidates and the measurement band,
- estimating (222; 312) the nature and/or the thickness of the material to be identified (M) from a comparison of the spectral coefficients determined with the spectral parameters of said at least one portion of the possible candidates, in said new common energy band.

2. Method for identifying a material (M1) according to claim 1, wherein the prior selecting (208$_1$, ..., 208$_p$; 308) of the possible candidates is carried out:

- either in at least one energy band that is common to all of the reference bands and to the measurement band (Bm),
- or in at least one energy band (NE$_{1,i}$) that is common to a predetermined portion of the reference bands (B$_{1,1}$, ..., B$_{1,N1}$) and to the measurement band (Bm).

3. Method for identifying a material (M) according to claim 1 or 2, wherein the reference materials and/or thicknesses comprise a plurality of reference materials each with a plurality of reference thicknesses.

4. Method for identifying a material (M) according to claim 3, wherein the estimation comprises the following steps, executed for each reference material:

- selecting (208$_1$, ..., 208$_p$; 308) a plurality of reference thicknesses for this reference material, referred to as possible candidates, by comparison of the spectral coefficients determined with the reference spectral parameters in at least one energy band (NE$_{1,i}$) that is common to the reference bands of each reference thickness (B$_{1,1}$, ..., B$_{1,N1}$) for this reference material and to the measurement band (Bm),
- selecting (212$_1$, ..., 212$_p$) two consecutive reference thicknesses for this reference material by comparison of the spectral coefficients determined with the spectral parameters of at least one portion of the possible candidates, in at least one energy band that is common to the reference bands of said at least one portion of possible candidates and to the measurement band (Bm),
- estimating (218$_1$, ..., 218$_p$) an optimum thickness for this reference material by comparison of the spectral coefficients determined with spectral parameters interpolated using spectral parameters relating to the two consecutive reference thicknesses selected, in at least one energy band (NE$_{1,j'}$) that is common to the reference bands of the two consecutive reference thicknesses selected (B$_{1,j}$, B$_{1,j+1}$) and to the measurement band (Bm).

5. Method for identifying a material (M) according to claim 4, wherein the estimation of the nature and/or of the thickness of the material to be identified comprises the selecting (222) of an optimum thickness, among all of the optimum thicknesses each associated with a reference material, on the basis of an optimization of a criterion evaluated in at least one energy band that is common to all of the energy bands wherein the optimum thicknesses were estimated.

6. Method for identifying a material (M) according to any of claims 1 to 5, wherein the reference spectral parameters relating to reference materials and/or thicknesses are probability density statistical parameters, each reference material and/or thickness being thus associated with a probability density making it possible to calculate a probability of measuring spectral coefficients in the presence of this reference material and/or thickness in its reference band.

7. Method for identifying a material (M) according to claim 6, wherein the comparison of the spectral coefficients determined with the reference spectral parameters relating to a reference material and/or thickness comprises the estimating of a value that takes the probability density of this reference material and/or thickness when the spectral coefficients determined are applied to it.

8. Method for identifying a material (M) according to claim 6, wherein the comparison of the spectral coefficients determined with the reference spectral parameters relating to a reference material and/or thickness comprises the estimation of a conditional posterior probability of being in the presence of this reference material and/or thickness knowing the determined spectral coefficients, on the basis of the product of a conditional posterior probability of measuring the spectral coefficients determined in the presence of this reference material and/or thickness and of a prior probability of being in the presence of this reference material and/or thickness, by application of Bayes' theorem.

9. Device for identifying a material (M) by spectral analysis of electromagnetic radiation able to pass through this material, comprising:

- a transmitter (10) of electromagnetic radiation,
- a device (12) for measuring a spectrum of electromagnetic radiation transmitted through the material to be identified,
- a calculator (20) programmed (28, 30, 32, 34) for:

  • determining (202, 204) at least one energy band (Bm), referred to as a measurement band, and spectral coefficients of a comparison function in this measurement band, using the measured spectrum, and
  • estimating (PH1, PH2; 306, 308, 310, 312), using the determined spectral coefficients, the nature and/or the thickness of the material to be identified (M) on the basis of a set of reference spectral parameters relating to reference materials and/or thicknesses, these reference spectral parameters being defined for each reference material and/or thickness in at least one energy band referred to as a reference band specific to each reference material and/or thickness,

**characterized in that** the calculator (20) is more precisely programmed for:

- prior selecting ($208_1$, ..., $208_p$; 308) of a plurality of reference materials and/or thicknesses, referred to as possible candidates, from a comparison of the spectral coefficients determined with at least one portion of the reference spectral parameters in a first common energy band that corresponds to the intersection of at least one portion of the reference bands and the measurement band,
- determining ($210_1$, ..., $210_p$; 310) at least one new common energy band that corresponds to the intersection of the reference bands of at least one portion of the possible candidates and the measurement band,
- estimating (222; 312) the nature and/or the thickness of the material to be identified (M) from a comparison of the spectral coefficients determined with the spectral parameters of said at least one portion of the possible candidates, in said new common energy band.

10. Computer program which can be downloaded from a communications network and/or recorded on a support that can be read by a computer and/or that can be executed by a processor, **characterized in that** it comprises instructions for the execution of the steps of a method for identifying a material (M) according to any of claims 1 to 8, when said program is executed on a computer.

## Figure 1

## Figure 2

```
┌─────────┐     ┌─────────┐     ┌─────────┐     ┌─────────┐
│   100   │ ──▶ │   102   │ ──▶ │   104   │ ──▶ │   106   │
└─────────┘     └─────────┘     └─────────┘     └─────────┘
```

## Figure 3

```
┌─────────┐     ┌─────────┐     ┌─────────┐
│   200   │ ──▶ │   202   │ ──▶ │   204   │
└─────────┘     └─────────┘     └─────────┘
```

```
        ┌─────────┐                              ┌─────────┐
        │  206₁   │                              │  206ₚ   │
        └─────────┘                              └─────────┘
             │                                        │
        ┌─────────┐                              ┌─────────┐
        │  208₁   │                              │  208ₚ   │
        └─────────┘                              └─────────┘
             │                                        │
        ┌─────────┐                              ┌─────────┐
        │  210₁   │                              │  210ₚ   │       PH1
        └─────────┘                              └─────────┘
             ┆              ● ● ●                     ┆
        ┌─────────┐                              ┌─────────┐
        │  212₁   │                              │  212ₚ   │
        └─────────┘                              └─────────┘
             │                                        │
        ┌─────────┐                              ┌─────────┐
        │  214₁   │                              │  214ₚ   │
        └─────────┘                              └─────────┘
             │                                        │
        ┌─────────┐                              ┌─────────┐
        │  216₁   │                              │  216ₚ   │
        └─────────┘                              └─────────┘
             │                                        │
        ┌─────────┐                              ┌─────────┐
        │  218₁   │                              │  218ₚ   │
        └─────────┘                              └─────────┘

                        ┌─────────┐
                        │   220   │                        PH2
                        └─────────┘
                             │
                        ┌─────────┐
                        │   222   │
                        └─────────┘
```

## *Figure 4*

## Figure 5

## Figure 6

**RÉFÉRENCES CITÉES DANS LA DESCRIPTION**

**Littérature non-brevet citée dans la description**

- **K. MEYER et al.** Qualitative and quantitative mixtures analysis by library search: infrared analysis of mixtures of carbohydrates. *Analytica Chimica Acta,* 01 Septembre 1993, vol. 281, 161-171